# EUROPEAN PATENT SPECIFICATION

(11) **EP 3 419 640 B1**
(45) Date of publication and mention of the grant of the patent: **07.09.2022**
(21) Application number: 16891809.2
(22) Date of filing: 24.02.2016
(51) Int. Cl.: A61K 31/192, A61K 31/7032

(54) **ADAPTOGENIC COMPOSITIONS AND APPLICATIONS THEREOF**
ADAPTOGENE ZUSAMMENSETZUNGEN UND ANWENDUNGEN DAVON
COMPOSITIONS ADAPTOGÈNES ET LEURS APPLICATIONS

(43) Date of publication of application: 02.01.2019
(73) Proprietor: Sami-Sabinsa Group Limited, Karnataka, Bangalore 560058 (IN)
(72) Inventor: MAJEED, Muhammed, East Windsor, NJ 08520 (US); NAGABHUSHANAM, Kalyanam., East Windsor, NJ 08520 (US)
(74) Representative: Dr. Gassner & Partner mbB
(86) International application number: PCT/US2016/019228
(87) International publication number: WO 2017/146690

(56) References cited:
- WO-A2-2012/142511
- US-A1- 2005 192 251
- US-A1- 2008 275 117
- US-A1- 2010 062 989
- US-A1- 2011 218 172
- US-A1- 2013 295 668

## Description

### FIELD OF INVENTION

The present invention in general relates to the adaptogenic activity of boswellic acids-polysaccharide compositions wherein the polysaccharide component is not less than 70% by weight of the said composition, derived from *Boswellia serrata* in combination with (i) the concentrate of the liquid endosperm of *Cocos nucifera* standardized to contain not less than 70% w/w of total dissolved solids or (ii) the extract of *Emblica officinalis* fruit standardized to contain 10% w/w and above of 1-O-galloyl-β-D-glucose (β-glucogallin) and not more than 5% w/w of gallic acid.

### BACKGROUND OF THE INVENTION

### Description of prior art

Composition comprising boswellic acids and polysaccharides obtained from *Boswellia serrata* and effects therein in terms of down-regulating pro-inflammatory cytokines has been disclosed by Muhammed Majeed et al in United States Patent Application 20110218172. Similar water-soluble bioactive fraction obtained from the gum resin exudate of *Boswellia serrata* enriched in polysaccharides having applications in anti-inflammatory and anti-arthritic management methods have also been disclosed in United States Patent Application 20050192251. In WO 2012/142511 A2, orthomolecular compositions and pharmaceutical formulations thereof that include multiple phytochemically-active nutraceutical compounds, and that possess potent anti-inflammatory and extracellular matrix-stabilizing properties in vitro and in vivo, are disclosed. Also disclosed are prophylactic and therapeutic methods, as well as dosing regimens and medicaments for use in preventing chronic disease, treating acute or long-term inflammatory-mediated conditions in affected or at-risk subjects, and stabilizing the mammalian extracellular matrix. US 2008/275117 A1 discloses a composition comprising alpha- and/or beta-boswellic acid and/or their C-acetates in an amount greater than 65% by weight. In addition, a method for making a boswellia species extract and a method of treating arthritis by administering therapeutically effective amount of the composition and a pharmaceutical carrier are disclosed. The present invention discloses adaptogenic activity of boswellic acids-polysaccharide compositions derived from *Boswellia serrata* in combination with (i) the concentrate of the liquid endosperm of *Cocos nucifera* standardized to contain not less than 70% w/w of total dissolved solids or (ii) the extract of *Emblica officinalis* fruit standardized to contain 10% w/w and above of 1-O-galloyl-β-D-glucose (β-glucogallin) and not more than 5% w/w of gallic acid.

While no specific definition of adaptogens exist, the term adaptogens or adaptogenic substances are stated to have the capacity to normalize body functions and strengthen systems compromised by stress. They are reported to have a protective effect on health against a wide variety of environmental assaults and emotional conditions. The role of adaptogens in phytotherapy is best summarized as follows. Most modern active substances are directed to well-defined clinical conditions. If preventive actions are intended, they are specific to a certain disease factor, e.g. vaccines, use of anti-virals, or they are directed to a certain pathological factor with a view to reducing the risk of disease, e.g. cholesterol lowering substances. In contrast to these approaches, the action of adaptogens is reported to be neither directed to eliminate the symptoms of already existing diseases nor is the action specific. If used in an already developed disease, adaptogens are thought to create unspecific effects and in this case they mostly are thought to prevent complications of a disease and to strengthen the general state of the organism. Adaptogens are described to promote non-specific resistance of the body against diseases and different types of stress. This is why a much broader spectrum of action is attributed to adaptogens as compared to most conventional active substances. Nevertheless, the concept of adaptogens is sufficient to be considered in the assessment of traditional herbal medicinal products (REFLECTION PAPER ON ADAPTOGENIC CONCEPT, COMMITTEE ON HERBAL MEDICINAL PRODUCTS (HMPC), European Medicines Agency, Evaluation of Medicines for Human Use, London, 8 May 2008, Doc. Ref. EMEA/HMPC/102655/2007). For example, the adaptogenic effect of ethanolic extract of *Bacopa monnieri* in acute stress mice models has been disclosed in Anju, "Bacopa monnieri - a Preliminary Study Evaluating Its Anti-Stress Activity in Swiss Albino Mice", Research Journal of Pharmaceutical, Biological and Chemical Sciences, Volume 2 Issue 4, October - December 2011, pages 786-794.

The importance of Nutrition and supplementation strategies focusing on adaptogenic effects (acute and chronic) like favorable body composition, physical performance, metabolism, endurance, recovery from stress and injury and the maintenance of body homeostasis form the crux of sports nutrition have also been reported in prior art (Dairy Australia, "SPORTS NUTRITION", Good Health and Nutrition, ABN 60 105 227 987 Level 5, IBM Tower, 60 City Road, Southbank Victoria 3006 Australia).

It is thus the principle objective of the present invention to disclose adaptogenic activity of boswellic acids-polysaccharide compositions derived from *Boswellia serrata* in combination with (i) the concentrate of the liquid endosperm of *Cocos nucifera* standardized to contain not less than 70% w/w of total dissolved solids or (ii) the extract of *Emblica officinalis* fruit standardized to contain 10% w/w and above of 1-O-galloyl-β-D-glucose (β-glucogallin) and not more than 5% w/w of gallic acid. The invention describes the tests for adaptogenic properties of said compositions.

The present invention fulfills the aforesaid objectives and provides further related advantages.

### SUMMARY OF THE INVENTION

The present invention discloses the adaptogenic activity of boswellic acids-polysaccharide compositions derived from *Boswellia serrata* in combination with (i) the concentrate of the liquid endosperm of *Cocos nucifera* standardized to contain not less than 70% w/w of total dissolved solids or (ii) the extract of *Emblica officinalis* fruit standardized to contain 10% w/w and above of 1-O-galloyl-β-D-glucose (β-glucogallin) and not more than 5% w/w of gallic acid.

Other features and advantages of the present invention will become apparent from the following more detailed description, taken in conjunction with the accompanying images, which illustrate, by way of example, the principle of the invention.

### BRIEF DESCRIPTION OF THE DRAWINGS

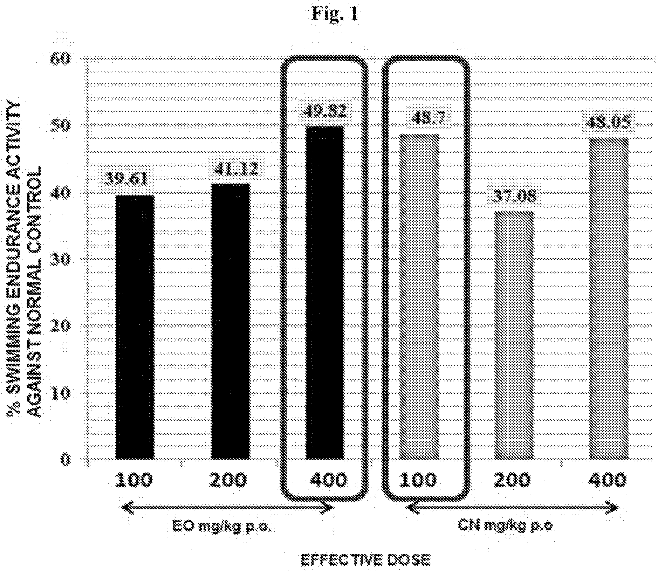

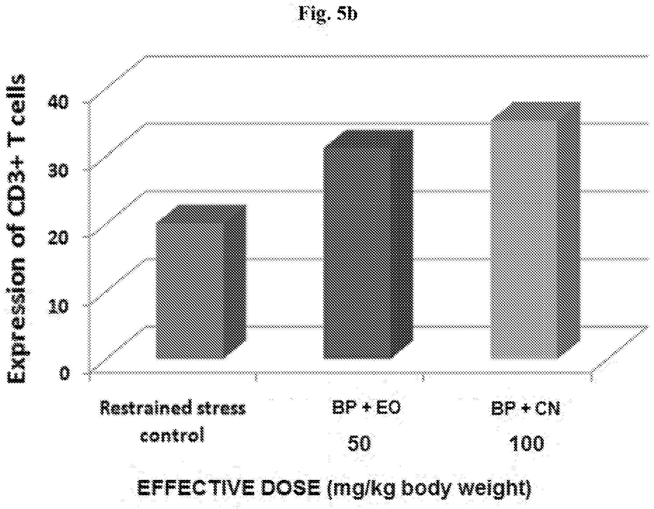

**Fig.1** is a graphical representation of the % enhancement in swimming endurance activity of test animals treated with 100, 200 and 400 mg/kg body weight per orally of concentrate of liquid endosperm of *Cocos nucifera* standardized to contain not less than 70% w/w of total dissolved solids (CN) or the extract of *Emblica officinalis* fruit standardized to contain 10% w/w and above of 1-O-galloyl-β-D-glucose (β-glucogallin) and not more than 5% w/w of gallic acid (EO) as measured in comparison to Normal Control animals.

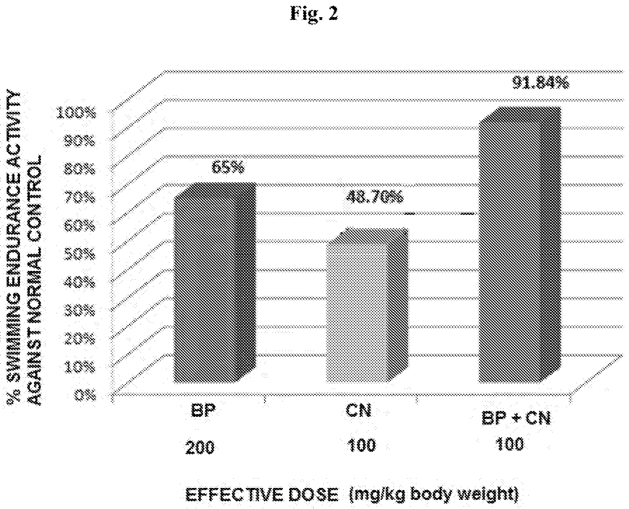

**Fig.2** is a graphical representation of % enhancement in swimming endurance activity of test animals treated with 100 mg/kg body weight per orally of combined ingredients comprising boswellic acids-polysaccharide (BP) compositions wherein the polysaccharide component is not less than 70% by weight of the said composition, derived from *Boswellia serrata* in combination with the concentrate of the liquid endosperm of *Cocos nucifera* standardized to contain not less than 70% w/w of total dissolved solids as compared with test animals treated with individual ingredients BP (200 mg/kg body weight) and CN (100 mg/kg body weight).

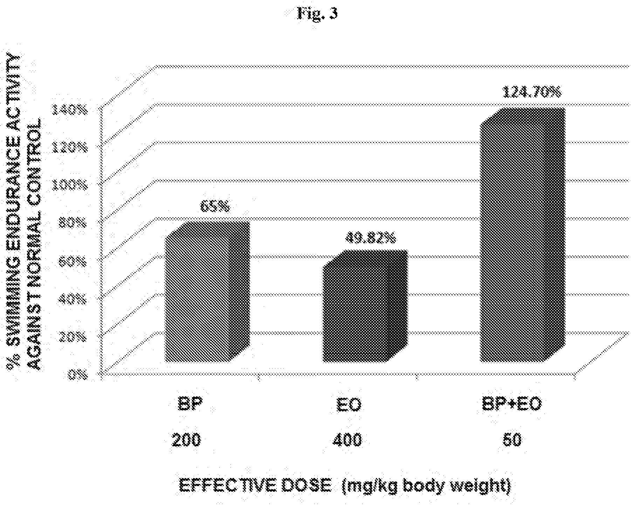

**Fig.3** is a graphical representation of % enhancement in swimming endurance activity of test animals treated with 50 mg/kg body weight boswellic acids-polysaccharide compositions wherein the polysaccharide component is not less than 70% by weight of the said composition, derived from *Boswellia serrata* in combination with the extract of *Emblica officinalis* fruit standardized to contain 10% w/w and above of 1-O-galloyl-β-D-glucose (β-glucogallin) and not more than 5% w/w of gallic acid as compared with test animals treated with individual ingredients BP (200 mg/kg body weight) and EO (50 mg/kg body weight).

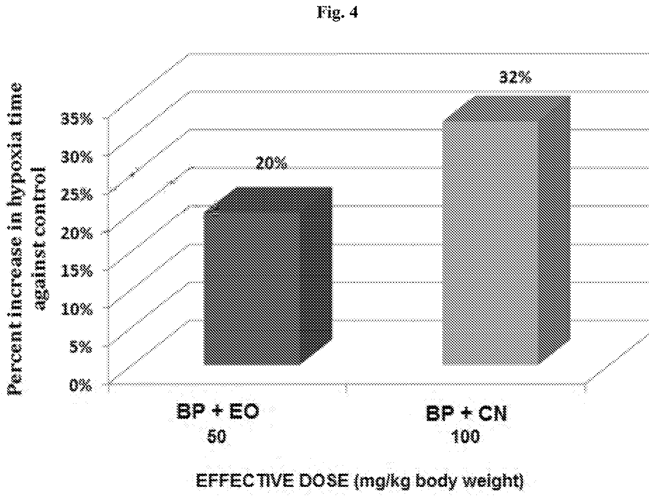

**Fig. 4** is graphical representation of % increase in hypoxia induction time against control, of test animals treated with 50 mg/kg body weight per orally of boswellic acids-polysaccharide compositions wherein the polysaccharide component is not less than 70% by weight of the said composition, derived from *Boswellia serrata* in combination with the extract of *Emblica officinalis* fruit standardized to contain 10% w/w and above of 1-O-galloyl-β-D-glucose (β-glucogallin) and not more than 5% w/w of gallic acid or 100 mg/kg body weight per orally of boswellic acids-polysaccharide compositions wherein the polysaccharide component is not less than 70% by weight of the said composition, derived from *Boswellia serrata* in combination with the concentrate of the liquid endosperm of *Cocos nucifera* standardized to contain not less than 70% w/w of total dissolved solids as compared with test animals treated with said ingredients alone during recovery against hypoxia.

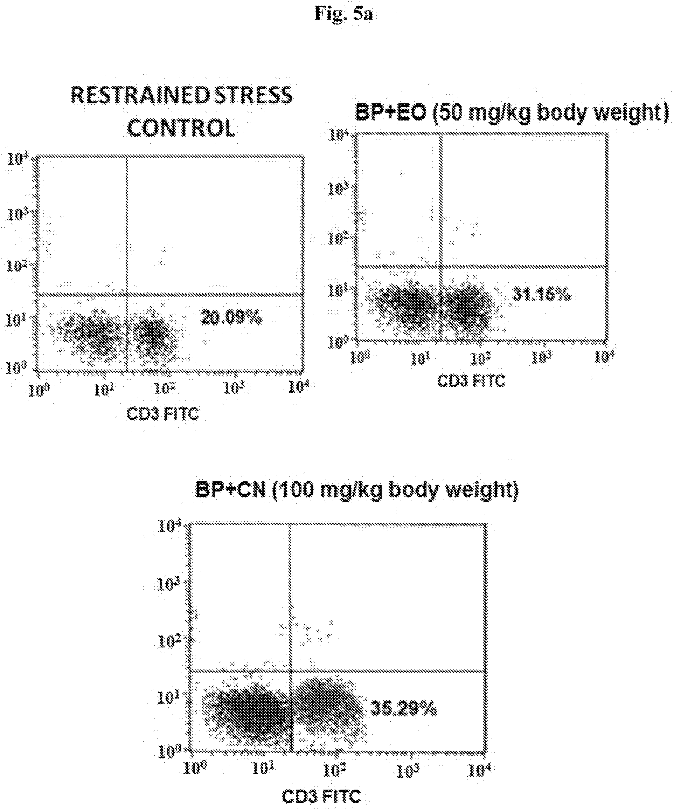

**Fig. 5a** shows the expression of increase in CD3+ T cell expression in experimental mice subjected to chronic restraint stress, said mice treated with
   (1) 50 mg/kg body weight per orally of boswellic acids-polysaccharide compositions wherein the polysaccharide component is not less than 70% by weight of the said composition, derived from *Boswellia serrata* in combination with the extract of *Emblica officinalis* fruit standardized to contain 10% w/w and above of 1-O-galloyl-β-D-glucose (β-glucogallin) and not more than 5% w/w of gallic acid;
   (2) 100 mg/kg body weight per orally of boswellic acids-polysaccharide compositions wherein the polysaccharide component is not less than 70% by weight of the said composition, derived from *Boswellia serrata* in combination with the concentrate of the liquid endosperm of *Cocos nucifera* standardized to contain not less than 70% w/w of total dissolved solids); the effect of compositions (1) and (2) herein above as compared with test mice treated with said ingredients alone. **Fig. 5b** is graphical representation of the effects represented in **Fig. 5a****.**

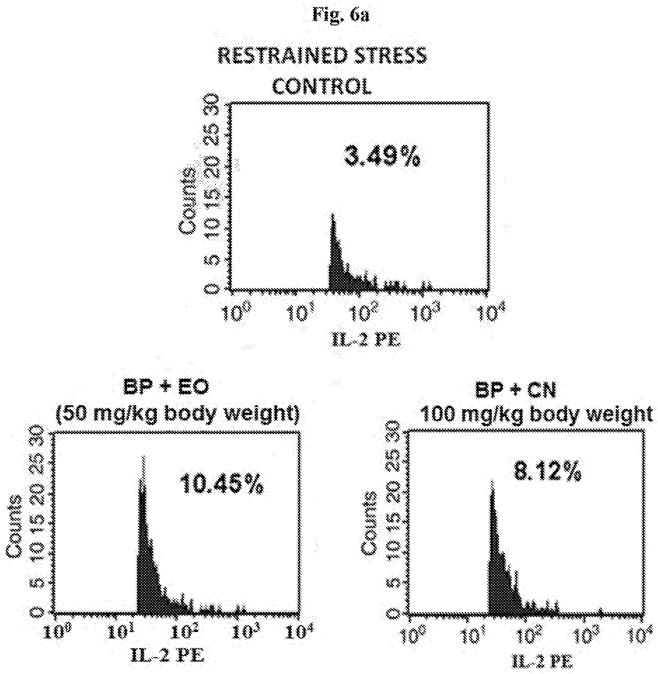

**Fig. 6a** shows the cytokine IL-2 expression in experimental mice subjected to chronic restraint stress, said mice treated with
   (1)50 mg/kg body weight per orally of boswellic acids-polysaccharide compositions wherein the polysaccharide component is not less than 70% by weight of the said composition, derived from *Boswellia serrata* in combination with the extract of *Emblica officinalis* fruit standardized to contain 10% w/w and above of 1-O-galloyl-β-D-glucose (β-glucogallin) and not more than 5% w/w of gallic acid (i) the concentrate of the liquid endosperm of *Cocos nucifera* standardized to contain not less than 70% w/w of total dissolved solids
   (2) 100 mg/kg body weight per orally of boswellic acids-polysaccharide compositions wherein the polysaccharide component is not less than 70% by weight of the said composition, derived from *Boswellia serrata* in combination with the concentrate of the liquid endosperm of *Cocos nucifera* standardized to contain not less than 70% w/w of total dissolved solids; the effect of compositions (1) and (2) herein above as compared with test mice treated with said ingredients alone.

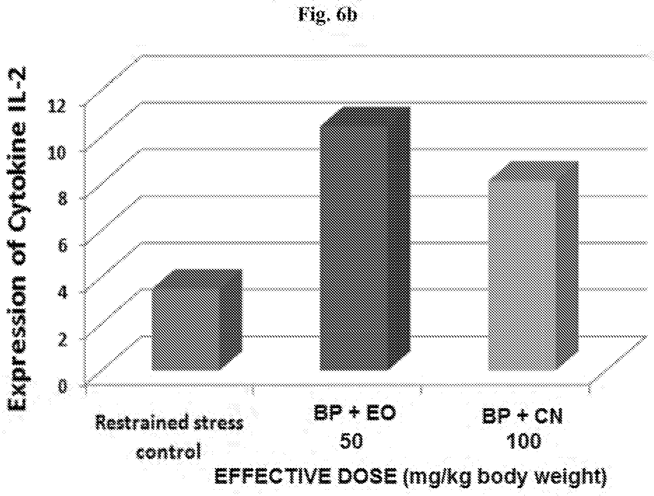

**Fig. 6b** is a graphical representation of the effects represented in **Fig. 6a****.**

### DESCRIPTION OF THE PREFERRED EMBODIMENTS (Figs.1-6)

In the most preferred embodiment, the present invention relates to a non-therapeutic method of increasing the physiological endurance of a mammal in the event of physical stress, said method comprising a step of orally administering an effective amount of a composition (adaptogenic composition) depending on the body weight of said mammal, wherein said composition includes,
a) Boswellic acids-polysaccharide (BP) compositions derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide compositions derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid.

In another most preferred embodiment, the present invention relates to a composition including,
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid for use in a method of protecting against hypoxic injury in a mammal during physical stress, said method comprising step of orally administering effective amount of the composition (adaptogenic composition) depending on the body weight of said mammal to bring about the effect of delayed induction of hypoxia.

In yet another most preferred embodiment, the present invention relates to a composition including,
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid for use in a method of treatment of stress induced suppression of cellular immunity in a mammal undergoing physical stress, said method comprising step of orally administering effective amount of the composition (adaptogenic composition) depending on the body weight of said mammal.

Furthermore, the present application discloses a method of increasing systemic interleukin-2 (IL-2) expression in mammals undergoing physical stress and stress induced suppression of cellular immunity, said method comprising step of orally administering effective amounts of compositions (adaptogenic compositions) depending on the body weight of said mammal, wherein said compositions include,
a) Boswellic acids-polysaccharide (BP) compositions derived from *Boswellia serrata* wherein the polysaccharide component is not less than 70% by weight of the said composition combined with the concentrate of the liquid endosperm of *Cocos nucifera* standardized to contain not less than 70% w/w of total dissolved solids;
b) Boswellic acids-polysaccharide compositions derived from *Boswellia serrata* wherein the polysaccharide component is not less than 70% by weight of the said composition combined with the extract of *Emblica officinalis* fruit standardized to contain 10% w/w and above of 1-O-galloyl-β-D-glucose (β-glucogallin) and not more than 5% w/w of gallic acid.

In yet another most preferred embodiment, the present invention relates to a non-therapeutic method of sustaining neuromuscular coordination in a mammal undergoing physical stress, said method comprising step of orally administering effective amount of composition (adaptogenic compositions) depending on the body weight of said mammal, wherein said composition includes, (a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
(b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid.

In yet another most preferred embodiment, the present invention also relates to a composition including,
(a) Boswellic acids-polysaccharide (BP) compositions derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
(b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid to bring about the effect of attenuating said stress induced atrophy of spleen and thymus glands and hypertrophy of adrenal glands for use in a method of treating stress induced immunosuppressive effects in spleen, thymus and adrenal glands of a mammal leading to undesirable atrophy or hypertrophy, said method comprising step of orally administering effective amount of the composition (adaptogenic composition) depending on the body weight of said mammal.

The aforesaid most preferred embodiments are elucidated herein below as illustrative examples.

The acute oral safety study was carried out following OECD guidelines No. 423. A single dose of the test material(s) BP, EO and CN was administered to a group of three females each up to a dose level of 2000 mg/kg. The animals were observed for any gross behavioural changes, for a total of 14 days. No change in general behaviour or any mortality was observed in groups of animals treated by different doses of the test material for 14 days. For the pharmacological studies, the Applicants tested graded doses ranging from 50mg/kg to 800mg/kg of the individual extracts BP, CN and EO to calculate the most effective dose for the individual extracts in the Swimming endurance test (procedure discussed herein below) [**Fig.1**]. The effective doses include BP: 200mg/kg, EO: 400mg/kg and CN: 100mg/kg. Combination of BP (200) + CN (100) was then tested at 50, 100 and 200 mg/kg for swimming endurance and the most effective dose of the combination was achieved at 100mg/kg. Similarly, Combination of BP (200) + EO (400) was then tested at 50, 100 and 200 mg/kg for swimming endurance and the most effective dose of the combination was achieved at 50mg/kg. The testing procedures are further described in detail in EXAMPLE I presented herein below.

### EXAMPLE I- SWIMMING ENDURANCE (endurance to physical stress) TEST (Figs.1, 2 and 3)

Animals: Male Swiss albino mice
Weight: 25-30 g
Number of animals per group: 06
Test groups are represented in the following table

| Test Group | Characterization |
|---|---|
| Group I (Normal control) | Without swimming stress |
| Group II | Swimming stress control group |
| Group III | (BP+CN)-Adaptogenic composition: 100 mg/kg body weight per oral administration |
| Group IV | (BP + EO)-Adaptogenic composition: 50 mg/kg body weight per oral administration |

Test materials BP, CN and EO formulated as adaptogenic compositions mentioned herein above (Group III and Group IV) were administered per orally to Swiss albino mice (25-30g) of either sex once a day for 14 days. On day 15, one hour after drug administration, the swimming time of each animal was measured individually by the Swimming Endurance Test. The animals were allowed to swim inside a perplex glass beaker (30 cm high with 20 cm diameter, containing water up to 25 cm height) maintained at 26 ± 1 °C. The mice were allowed to swim till they got exhausted which was considered as the endpoint. The mean swimming time for each group was calculated **(****Fig.2** **for group III and** **Fig. 3** **for group IV).** Both (BP+CN)-Adaptogenic composition: 100 mg/kg body weight per oral administration (Group III) and (BP + EO)-Adaptogenic composition: 50 mg/kg body weight per oral administration (Group IV) were very effective in increasing the endurance capacity of test animals in the Swimming Endurance test.

### EXAMPLE II A-Anti-fatigue effect

Animals: Swiss albino mice of either sex
Weight: 25-30 g
No. of animals per group: 10

Test groups included the following.

| Group I (Normal control) | Without swimming stress |
|---|---|
| Group II | Swimming stress control group |
| Group III | (BP+CN)-Adaptogenic composition: 100 mg/kg body weight per oral administration |
| Group IV | (BP + EO)-Adaptogenic composition: 50 mg/kg body weight per oral administration |

Test material was administered orally once a day for 14 days to animal groups **III** and **IV**. On day 15, one hour after drug administration in groups **III** and **IV**, the animals were allowed to swim inside a perplex glass beaker (30 cm high with 20 cm diameter, containing water up to 25 cm height) maintained at 26 ± 1 °C. The mice were allowed to swim till they got exhausted which was considered as the endpoint. Group I animals were not exposed to the swimming stress test. Group II animals were exposed to the swimming endurance test as mentioned above without being administered test materials. Only pre-trained test mice which stayed on a rotating rod at 20 rpm, for more than 5 minutes in three successive trials for 5 consecutive days, were used in this study. On day 15, the animals of Group II, III and IV that underwent swimming stress were immediately taken out, dried with tissue paper and placed on the rotating rod to monitor anti-fatigue and motor coordination effects. The number of mice that stayed on the rota-rod for 180 seconds or more were considered as un tired with motor coordination. The percent effect of each group was calculated on the basis of the number of mice that stayed on the rota- rod for > 180 seconds (by all or non- method). The same animals were again placed on the rota- rod after 30 minutes of removal from the swimming bath, to monitor the anti fatigue effect once again. Similarly, the animals of Group I which were not allowed to swim were also placed on the rotating rod to see the anti fatigue effect in normal animals. The results of the Anti-fatigue effect testing (neuromuscular coordination) are represented below in **Table A.**

**Table A**

| **TREATMENT** | **No of animals that stayed on the rod for > 180 seconds/total no of animals in the group** |
|---|---|
| Group I (No stress control) without any test material treatment | 6/10 |
| Group II (stress control) without any test material treatment | 2/10 |
| Group III (BP+CN)-Adaptogenic composition: 100 mg/kg body weight per oral administration | 8/10 |
| Group IV (BP + EO)-Adaptogenic composition: 50 mg/kg body weight per oral administration | 7/10 |

### EXAMPLE 2B- Measurement of Hypoxia time (Fig. 4)

3 groups of test animals (Swiss albino mice of either sex; Weight: 25-30 g and number of animals per group: 10) where Group I includes animals without any test material treatment, Group II animals administered (BP+CN)-Adaptogenic composition: 100 mg/kg body weight per oral administration for 15 days and Group III animals administered (BP + EO)-Adaptogenic composition: 50 mg/kg body weight per oral administration animals for 15 days were tested for their ability for resist hypoxia induction. On day 15, one hour after treatment, the hypoxia time was recorded individually or each animal by placing the animal in an empty glass jar of 300-ml capacity attached to an electronic watch. The jars were made air-tight with greased glass stoppers and the time until onset of convulsion was recorded as the end point. The results of the hypoxia induction time testing are represented in Fig. 4. The graphs indicate that percentage hypoxia induction time was enhanced for Group II (32%) and Group III (20%) as compared to control untreated Group I.

### EXAMPLE 3-Chronic restraint stress test

Male Swiss albino mice, 10-12 weeks old and weighing about 20-22 grams were employed for this study. Mice were restrained in these 50ml conical polypropylene tubes for 12 h during the dark cycle (2000-0800 h) for 14 days. Experimental animals were divided into groups of eight animals each. Group-1 served restraint stress control group without treatment with test material. Group-II included animals treated with (BP + EO)-Adaptogenic composition: 50 mg/kg body weight per oral administration for 15 days and subjected to the chronic restraint stress test as described before. Group III included animals treated (BP+CN)-Adaptogenic composition: 100 mg/kg body weight per oral administration. Following the chronic restraint stress procedure, lymphocyte immunotyping to evaluate suppression of cellular immunity due to stress was done. Blood was taken from the retro-orbital plexus of animals from all the groups for the assessment of various immune cells surface markers. Murine anti-CD3+ monoclonal antibodies were used in a multi parametric flowcytometric assay to quantify the lymphocyte subsets associated with the cell-mediated immune response. These flourochrome labeled monoclonal antibodies were added directly to 100 µl of whole blood, which was then lysed using whole blood lysing reagent (BD Biosciences). Following the final centrifugation, samples were resuspended in phosphate buffer saline (pH, 7.4) and analyzed directly on the flowcytometer (BD Biosciences) using Cell Quest Pro Software (BD Biosciences) **(****Fig. 5a). Fig. 5a** shows 20.09% CD3+ T lymphocyte subsets in the restrained stress control group. Group II and III treated groups show 31.15% and 35.29% CD3+ T lymphocyte subsets indicating recovery of cellular immunity. Further, intracellular cytokine IL-2 levels were estimated in blood by flowcytometry using Phycoerythrin (PE) labeled IL-2 monoclonal antibodies. Acquisition and the analysis were done directly on flowcytometer using Cell Quest Pro software (BD Biosciences). Chronic restrained stress condition suppressed the expression of IL-2 to 3.49% in Group I- restraint stress control group. The % IL-2 expression increased in Groups II and III to 10.45% and 8.12%.

### EXAMPLE IV- BODY AND ORGAN WEIGHTS

After the last stress session, the body weights of all the animals from all the groups were taken following which the animals were sacrificed and their thymus, adrenal glands, and spleen, were removed and weighed. **Table B indicates that** (BP+CN)-Adaptogenic composition: 100 mg/kg body weight per oral administration and (BP+EO)- Adaptogenic composition: 50 mg/kg body weight per oral administration attenuated stress induced atrophy of spleen and thymus glands and hypertrophy of adrenal glands.

**(Table B)**

| TREATMENT | Thymus (mg) | Spleen (mg) | Adrenal glands (mg) |
|---|---|---|---|
| Normal control | 650.28±1.2 | 720.12±1.1 | 13.60±0.9 |
| Restraint Stress control | 310.29±1.2 | 390.25±1.9 | 38.65±1.3 |
| (BP+CN)-Adaptogenic composition: 100 mg/kg body weight per oral administration | 418.45±1.4 (34.85↓) | 456.72±1.9 (17.03↑) | 29.35±2.4 (24.06↓) |
| (BP+EO)- Adaptogenic composition: 50 mg/kg body weight per oral administration | 480.11±2.1 (53.75↑) | 530.21±2.2 (35.86↑) | 23.52±1.2 (39.14↓) |

The scope of the invention is to be interpreted only in conjunction with the appended claims.

## Claims

1. A method of increasing the physiological endurance of a mammal in the event of sustained physical activity, said method comprising a step of orally administering an effective amount of a composition (adaptogenic composition) depending on the body weight of said mammal, wherein said composition includes,
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid,
wherein the method does not comprise a method for treatment of the human or animal body by therapy.

2. Composition including
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid for use in a method of protecting against hypoxic injury in a mammal during physical stress, said method comprising step of orally administering an effective amount of the composition (adaptogenic composition) depending on the body weight of said mammal to bring about the effect of delayed induction of hypoxia.

3. Composition including
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid for use in a method of treatment of stress induced suppression of cellular immunity in a mammal undergoing physical stress, said method comprising a step of orally administering an effective amount of the composition (adaptogenic composition) depending on the body weight of said mammal.

4. A method of sustaining neuromuscular coordination in a mammal undergoing physical stress, said method comprising a step of orally administering an effective amount of a composition (adaptogenic composition) depending on the body weight of said mammal, wherein said composition includes,
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid,
wherein the method does not comprise a method for treatment of the human or animal body by therapy.

5. Composition including
a) Boswellic acids-polysaccharide (BP) composition derived from *Boswellia serrata* wherein the polysaccharide component is not less than 70% by weight of the said composition combined with a concentrate of the liquid endosperm of *Cocos nucifera* standardized to contain not less than 70% w/w of total dissolved solids;
or
b) Boswellic acids-polysaccharide composition derived from *Boswellia serrata* wherein the polysaccharide component is not less than 70% by weight of the said composition combined with an extract of *Emblica officinalis* fruit standardized to contain 10% w/w and above of 1-O-galloyl-β-D-glucose (β-glucogallin) and not more than 5% w/w of gallic acid to bring about the effect of attenuating said stress induced atrophy of spleen and thymus glands and hypertrophy of adrenal glands
for use in a method of treating stress induced immunosuppressive effects in spleen, thymus and adrenal glands of a mammal leading to undesirable atrophy or hypertrophy, said method comprising a step of orally administering an effective amount of the composition (adaptogenic composition) depending on the body weight of said mammal.

## Patentansprüche

1. Verfahren zum Erhöhen der physiologischen Ausdauer eines Säugers im Falle anhaltender körperlicher Aktivität, wobei das Verfahren einen Schritt der oralen Verabreichung einer wirksamen Menge einer Zusammensetzung (adaptogene Zusammensetzung) in Abhängigkeit vom Körpergewicht des Säugers umfasst, wobei die Zusammensetzung beinhaltet,
a) Boswelliasäuren-Polysaccharid (BP)-Zusammensetzung, die von *Boswellia serrata* abgeleitet ist, wobei der Polysaccharid-Bestandteil nicht weniger als 70 Gew.-% der Zusammensetzung beträgt, kombiniert mit einem Konzentrat des flüssigen Endosperms von *Cocos nucifera,* das so standardisiert ist, dass es nicht weniger als 70 Gew.-% der gesamten gelösten Feststoffe enthält;
oder
b) Boswelliasäuren-Polysaccharid-Zusammensetzung, die von *Boswellia serrata* abgeleitet ist, wobei der Polysaccharid-Bestandteil nicht weniger als 70 Gew.-% der Zusammensetzung beträgt, kombiniert mit einem Extrakt der *Emblica* officinalis-Frucht, der so standardisiert ist, dass er 10 Gew.-% und mehr an 1-O-Galloyl-β-D-Glucose (β-Glucogallin) und nicht mehr als 5 Gew.-% an Gallussäure enthält, wobei das Verfahren kein Verfahren zur Behandlung des menschlichen oder tierischen Körpers durch Therapie umfasst.

2. Zusammensetzung beinhaltend
a) Boswelliasäuren-Polysaccharid (BP)-Zusammensetzung, die von *Boswellia serrata* abgeleitet ist, wobei der Polysaccharid-Bestandteil nicht weniger als 70 Gew.-% der Zusammensetzung beträgt, kombiniert mit einem Konzentrat des flüssigen Endosperms von *Cocos nucifera,* das so standardisiert ist, dass es nicht weniger als 70 Gew.-% der gesamten gelösten Feststoffe enthält;
oder
b) Boswelliasäuren-Polysaccharid-Zusammensetzung, die von *Boswellia serrata* abgeleitet ist, wobei der Polysaccharid-Bestandteil nicht weniger als 70 Gew.-% der Zusammensetzung beträgt, kombiniert mit einem Extrakt der *Emblica* officinalis-Frucht, der so standardisiert ist, dass er 10 Gew.-% und mehr an 1-O-Galloyl-β-D-Glucose (β-Glucogallin) und nicht mehr als 5 Gew.-% an Gallussäure enthält
zur Verwendung in einem Verfahren zum Schutz gegen hypoxische Schädigung bei einem Säuger während körperlicher Belastung, wobei das Verfahren einen Schritt der oralen Verabreichung einer wirksamen Menge der Zusammensetzung (adaptogene Zusammensetzung) in Abhängigkeit vom Körpergewicht des Säugers umfasst, um die Wirkung einer verzögerten Induktion von Hypoxie zu bewirken.

3. Zusammensetzung beinhaltend
a) Boswelliasäuren-Polysaccharid (BP)-Zusammensetzung, die von *Boswellia serrata* abgeleitet ist, wobei der Polysaccharid-Bestandteil nicht weniger als 70 Gew.-% der Zusammensetzung beträgt, kombiniert mit einem Konzentrat des flüssigen Endosperms von *Cocos nucifera,* das so standardisiert ist, dass es nicht weniger als 70 Gew.-% der gesamten gelösten Feststoffe enthält;
oder
b) Boswelliasäuren-Polysaccharid-Zusammensetzung, die von *Boswellia serrata* abgeleitet ist, wobei der Polysaccharid-Bestandteil nicht weniger als 70 Gew.-% der Zusammensetzung beträgt, kombiniert mit einem Extrakt der *Emblica* officinalis-Frucht, der so standardisiert ist, dass er 10 Gew.-% und mehr an 1-O-Galloyl-β-D-Glucose (β-Glucogallin) und nicht mehr als 5 Gew.-% an Gallussäure enthält
zur Verwendung in einem Verfahren zur Behandlung von belastungsinduzierter Unterdrückung zellulärer Immunität in einem Säuger, der körperlicher Belastung ausgesetzt ist, wobei das Verfahren einen Schritt der oralen Verabreichung einer wirksamen Menge der Zusammensetzung (adaptogene Zusammensetzung) in Abhängigkeit vom Körpergewicht des Säugers umfasst.

4. Verfahren zum Unterstützen der neuromuskulären Koordination bei einem Säuger, der körperlicher Belastung ausgesetzt ist, wobei das Verfahren einen Schritt der oralen Verabreichung einer wirksamen Menge einer Zusammensetzung (adaptogene Zusammensetzung) in Abhängigkeit vom Körpergewicht des Säugers umfasst, wobei die Zusammensetzung beinhaltet,
a) Boswelliasäuren-Polysaccharid (BP)-Zusammensetzung, die von *Boswellia serrata* abgeleitet ist, wobei der Polysaccharid-Bestandteil nicht weniger als 70 Gew.-% der Zusammensetzung beträgt, kombiniert mit einem Konzentrat des flüssigen Endosperms von *Cocos nucifera,* das so standardisiert ist, dass es nicht weniger als 70 Gew.-% der gesamten gelösten Feststoffe enthält;
oder
b) Boswelliasäuren-Polysaccharid-Zusammensetzung, die von *Boswellia serrata* abgeleitet ist, wobei der Polysaccharid-Bestandteil nicht weniger als 70 Gew.-% der Zusammensetzung beträgt, kombiniert mit einem Extrakt der *Emblica* officinalis-Frucht, der so standardisiert ist, dass er 10 Gew.-% und mehr an 1-O-Galloyl-β-D-Glucose (β-Glucogallin) und nicht mehr als 5 Gew.-% an Gallussäure enthält,
wobei das Verfahren kein Verfahren zur Behandlung des menschlichen oder tierischen Körpers durch Therapie umfasst.

5. Zusammensetzung beinhaltend
a) Boswelliasäuren-Polysaccharid (BP)-Zusammensetzung, die von *Boswellia serrata* abgeleitet ist, wobei der Polysaccharid-Bestandteil nicht weniger als 70 Gew.-% der Zusammensetzung beträgt, kombiniert mit einem Konzentrat des flüssigen Endosperms von *Cocos nucifera,* das so standardisiert ist, dass es nicht weniger als 70 Gew.-% der gesamten gelösten Feststoffe enthält;
oder
b) Boswelliasäuren-Polysaccharid-Zusammensetzung, die von *Boswellia serrata* abgeleitet ist, wobei der Polysaccharid-Bestandteil nicht weniger als 70 Gew.-% der Zusammensetzung beträgt, kombiniert mit einem Extrakt der *Emblica* officinalis-Frucht, der so standardisiert ist, dass er 10 Gew.-% und mehr an 1-O-Galloyl-β-D-Glucose (β-Glucogallin) und nicht mehr als 5 Gew.-% an Gallussäure enthält, um die Wirkung der Abschwächung der belastungsinduzierten Atrophie der Milz und der Thymusdrüsen und der Hypertrophie der Nebennieren zu bewirken
zur Verwendung in einem Verfahren zum Behandeln von belastungsinduzierten immunsuppressiven Effekten in Milz, Thymus und Nebennieren eines Säugers, die zu unerwünschter Atrophie oder Hypertrophie führen, wobei das Verfahren einen Schritt der oralen Verabreichung einer wirksamen Menge der Zusammensetzung (adaptogene Zusammensetzung) in Abhängigkeit vom Körpergewicht des Säugers umfasst.

## Revendications

1. Procédé d'augmentation de l'endurance physiologique d'un mammifère en cas d'activité physique soutenue, ledit procédé comprenant une étape consistant à administrer par voie orale une quantité efficace d'une composition (composition adaptogène) en fonction du poids corporel dudit mammifère, dans lequel ladite composition inclut
a) une composition d'acides boswelliques-polysaccharide (BP) dérivée de Boswellia serrata, dans laquelle le composant de polysaccharide n'est pas moins de 70 % en poids de ladite composition combiné à un concentré de l'endosperme liquide de Cocos nucifera standardisé pour contenir pas moins de 70 % p/p de solides dissous totaux ;
ou
b) une composition d'acides boswelliques-polysaccharide dérivée de *Boswellia serrata,* dans laquelle le composant de polysaccharide n'est pas moins de 70 % en poids de ladite composition combiné à un extrait de fruit *d'Emblica officinalis* standardisé pour contenir 10 % p/p et plus de 1-O-galloyl-β-D-glucose (β-glucogalline) et pas plus de 5 % p/p d'acide gallique,
dans lequel le procédé ne comprend pas de procédé de traitement du corps humain ou animal par thérapie.

2. Composition incluant
a) une composition d'acides boswelliques-polysaccharide (BP) dérivée de Boswellia serrata, dans laquelle le composant de polysaccharide n'est pas moins de 70 % en poids de ladite composition combiné à un concentré de l'endosperme liquide de Cocos nucifera standardisé pour contenir pas moins de 70 % p/p de solides dissous totaux ;
ou
b) une composition d'acides boswelliques-polysaccharide dérivée de Boswellia serrata, dans laquelle le composant de polysaccharide n'est pas moins de 70 % en poids de ladite composition combiné à un extrait de fruit d'Emblica officinalis standardisé pour contenir 10 % p/p et plus de 1-O-galloyl-β-D-glucose (β-glucogalline) et pas plus de 5 % p/p d'acide gallique,
destinée à être utilisée dans un procédé de protection contre la lésion hypoxique chez un mammifère au cours d'un stress physique, ledit procédé comprenant une étape consistant à administrer par voie orale une quantité efficace de la composition (composition adaptogène) en fonction du poids corporel dudit mammifère pour entraîner l'effet d'induction retardée d'hypoxie.

3. Composition incluant
a) une composition d'acides boswelliques-polysaccharide (BP) dérivée de Boswellia serrata, dans laquelle le composant de polysaccharide n'est pas moins de 70 % en poids de ladite composition combiné à un concentré de l'endosperme liquide de Cocos nucifera standardisé pour contenir pas moins de 70 % p/p de solides dissous totaux ;
ou
b) une composition d'acides boswelliques-polysaccharide dérivée de Boswellia serrata, dans laquelle le composant de polysaccharide n'est pas moins de 70 % en poids de ladite composition combiné à un extrait de fruit d'Emblica officinalis standardisé pour contenir 10 % p/p et plus de 1-O-galloyl-β-D-glucose (β-glucogalline) et pas plus de 5 % p/p d'acide gallique,
destinée à être utilisée dans un procédé de traitement de la suppression induite par le stress de l'immunité cellulaire chez un mammifère subissant un stress physique, ledit procédé comprenant une étape consistant à administrer par voie orale une quantité efficace de la composition (composition adaptogène) en fonction du poids corporel dudit mammifère.

4. Procédé de soutien de la coordination neuromusculaire chez un mammifère subissant un stress physique, ledit procédé comprenant une étape consistant à administrer par voie orale une quantité efficace d'une composition (composition adaptogène) en fonction du poids corporel dudit mammifère, dans lequel ladite composition inclut
a) une composition d'acides boswelliques-polysaccharide (BP) dérivée de Boswellia serrata, dans laquelle le composant de polysaccharide n'est pas moins de 70 % en poids de ladite composition combiné à un concentré de l'endosperme liquide de Cocos nucifera standardisé pour contenir pas moins de 70 % p/p de solides dissous totaux ;
ou
b) une composition d'acides boswelliques-polysaccharide dérivée de Boswellia serrata, dans laquelle le composant de polysaccharide n'est pas moins de 70 % en poids de ladite composition combiné à un extrait de fruit d'Emblica officinalis standardisé pour contenir 10 % p/p et plus de 1-O-galloyl-β-D-glucose (β-glucogalline) et pas plus de 5 % p/p d'acide gallique,
dans lequel le procédé ne comprend pas de procédé de traitement du corps humain ou animal par thérapie.

5. Composition incluant
a) une composition d'acides boswelliques-polysaccharide (BP) dérivée de Boswellia serrata, dans laquelle le composant de polysaccharide n'est pas moins de 70 % en poids de ladite composition combiné à un concentré de l'endosperme liquide de Cocos nucifera standardisé pour contenir pas moins de 70 % p/p de solides dissous totaux ; ou
b) une composition d'acides boswelliques-polysaccharide dérivée de Boswellia serrata, dans laquelle le composant de polysaccharide n'est pas moins de 70 % en poids de ladite composition combiné à un extrait de fruit d'Emblica officinalis standardisé pour contenir 10 % p/p et plus de 1-O-galloyl-β-D-glucose (β-glucogalline) et pas plus de 5 % p/p d'acide gallique pour entraîner l'effet d'atténuation de ladite atrophie induite par le stress de la rate et des glandes thymus et l'hypertrophie des glandes surrénales,
destinée à être utilisée dans un procédé de traitement des effets immunosuppresseurs induits par le stress dans la rate, le thymus et les glandes surrénales d'un mammifère conduisant à une atrophie ou hypertrophie indésirable, ledit procédé comprenant une étape consistant à administrer par voie orale une quantité efficace de la composition (composition adaptogène) en fonction du poids corporel dudit mammifère.
